# EUROPEAN PATENT SPECIFICATION

(11) **EP 2 214 738 B1**
(45) Date of publication and mention of the grant of the patent: **02.05.2012**
(21) Application number: 08845489.7
(22) Date of filing: 31.10.2008
(51) Int. Cl.: A61L 31/02, A61L 31/08, A61L 31/16, A61L 31/14

(54) **DEGRADABLE ENDOPROSTHESIS**
ABBAUBARE ENDOPROTHESE
ENDOPROTHÈSE VASCULAIRE DÉGRADABLE

(30) Priority: 02.11.2007 US 984960 P
(43) Date of publication of application: 11.08.2010
(73) Proprietor: Boston Scientific Limited, Christ Church (BB)
(72) Inventor: MILLER, Matthew, Stillwater Minnesota 55082 (US); WEBER, Jan, NL-6228 GJ Maastricht (NL)
(74) Representative: Peterreins, Frank
(86) International application number: PCT/US2008/081910
(87) International publication number: WO 2009/059081

(56) References cited:
- WO-A1-03/045582
- WO-A2-2009/018035
- WO-A2-2009/018037
- US-A1- 2006 193 886

## Description

This application claim benefit from U.S. Provisional Application No. 60/984,960, filed November 2, 2007, and now abandoned.

### TECHNICAL FIELD

This invention relates to medical devices, such as endoprostheses, and methods of making and using the same.

### BACKGROUND

The body includes various passageways including blood vessels such as arteries, and other body lumens. These passageways sometimes become occluded or weakened. For example, they can be occluded by a tumor, restricted by plaque, or weakened by an aneurysm. When this occurs, the passageway can be reopened or reinforced, or even replaced, with a medical endoprosthesis. An endoprosthesis is an artificial implant that is typically placed in a passageway or lumen in the body. Many endoprostheses are tubular members, examples of which include stents, stent-grafts, and covered stents.

Many endoprostheses can be delivered inside the body by a catheter. Typically the catheter supports a reduced-size or compacted form of the endoprosthesis as it is transported to a desired site in the body, for example the site of weakening or occlusion in a body lumen. Upon reaching the desired site, the endoprosthesis is installed so that it can contact the walls of the lumen. Stent delivery is further discussed in Heath, U.S. Patent No. 6,290,721 and in U.S. Patent Application Publication US2006/0193886, which discloses a medical device comprising one or more layers of porous metal on the surface of said device. The porous layers are generated by the process of dealloying in which a sacrificial material is selectively removed from a precursor alloy on the medical device. It results in the formation of a porous layer comprising an interstitial space, which is adapted to receive and release a therapeutic agent.

One method of installation involves expanding the endoprosthesis. The expansion mechanism used to install the endoprosthesis may include forcing it to expand radially. For example, the expansion can be achieved with a catheter that carries a balloon in conjunction with a balloon-expandable endoprosthesis reduced in size relative to its final form in the body. The balloon is inflated to deform and/or expand the endoprosthesis in order to fix it at a predetermined position in contact with the lumen wall. The balloon can then be deflated, and the catheter withdrawn.

Passageways containing endoprostheses can become re-occluded. Re-occlusion of such passageways is known as restenosis. It has been observed that certain drugs can inhibit the onset of restenosis when the drug is contained in the endoprosthesis. It is sometimes desirable for an endoprosthesis-contained therapeutic agent, or drug, to elute into the body fluid in a predetermined manner once the endoprosthesis is implanted.

It is also sometimes desirable for an implanted endoprosthesis to erode over time within the passageway. For example, a fully erodible endoprosthesis does not remain as a permanent object in the body, which may help the passageway recover to its natural condition. Erodible endoprostheses can be formed from, e.g., a polymeric material, such as polylactic acid, or from a metallic material, such as magnesium, iron, or an alloy thereof.

### SUMMARY

In one aspect, the document features an endoprosthesis that includes a body that has a first bioerodible metal and a surface, and a capsule formed of a second bioerodible metal disposed on the surface. The capsule includes a porous peripheral region and a drug-containing core.

In another aspect, the document features a method of forming an endoprosthesis, including applying a sacrificial template to a surface; applying a plurality of metal nanoclusters over the template; removing the sacrificial template to form a cavity defined by the metal nanoclusters; and loading drugs into the cavity.

Embodiments may include one or more of the following additional features. The first metal is selected from magnesium, iron, zinc, aluminum, calcium, tungsten, and alloys thereof. The second metal is selected from magnesium, iron, zinc, aluminum, calcium, tungsten, and alloys thereof. The first and second bioerodible metals are the same. The endoprosthesis is free of polymer. The porous peripheral region of the capsule comprises a plurality of interconnected pores having a width of about 0.5 nm to about 100 nm. The porous peripheral region has a porosity of about 70% or less. The capsule has the shape of a hollow sphere. The hollow sphere has an outer diameter of about 20 nm to about 10 µm. The hollow sphere has an inner diameter of about 10 nm to about 5 µm. The porous peripheral region has a thickness of about 1 nm to about 1 µm.

Embodiments may also include one or more of the following additional features. The method further comprises applying the plurality of metal nanoclusters comprising bioerodible metal. The method further comprises applying a polyelectrolyte layer to the surface before applying the sacrificial template over the polyelectrolyte layer. The method further comprises removing the polyelectrolyte layer and the sacrificial template by heating, UV light exposure, or dissolution. The method comprises applying the polyelectrolyte layer and the sacrificial template by LBL deposition. The sacrificial template is a polymer particle. The polymer particle has a size of about 10 nm to about 10 µm. The sacrificial template is a sphere. The sacrificial template is pretreated with a polyelectrolyte to be encapsulated by a polyelectrolyte coating. The method comprises applying the plurality of metal nanoclusters in an electrical field. The method comprises applying the drug by soaking the endoprosthesis in a solution having the drug. The plurality of metal nanoclusters form a first porous coating. The method further comprises forming a second coating over the first coating by applying a second plurality of metal nanoclusters. The second coating has a relatively lower porosity than the first coating and/or the second coating has a relatively smaller pore size than the first coating. The second coating is nonporous.

An erodible or bioerodible endoprosthesis, e.g., a stent, refers to a device, or a portion thereof, that exhibits substantial mass or density reduction or chemical transformation, after it is introduced into a patient, e.g., a human patient. Mass reduction can occur by, e.g., dissolution of the material that forms the device and/or fragmenting of the device. Chemical transformation can include oxidation/reduction, hydrolysis, substitution, electrochemical and/or addition reactions, or other chemical reactions of the material from which the device, or a portion thereof, is made. The erosion can be the result of a chemical and/or biological interaction of the device with the body environment, e.g., the body itself or body fluids, into which it is implanted and/or erosion can be triggered by applying a triggering influence, such as a chemical reactant or energy to the device, e.g., to increase a reaction rate. For example, a device, or a portion thereof, can be formed from an active metal, e.g., Mg or Ca or an alloy thereof, and which can erode by reaction with water, producing the corresponding metal oxide and hydrogen gas (a redox reaction). For example, a device, or a portion thereof, can be formed from an erodible or bioerodible polymer, or an alloy or blend of erodible or bioerodible polymers which can erode by hydrolysis with water. The erosion occurs to a desirable extent in a time frame that can provide a therapeutic benefit. For example, in embodiments, the device exhibits substantial mass reduction after a period of time, following which a function of the device, such as support of the lumen wall or drug delivery, is no longer needed or desirable. In particular embodiments, the device exhibits a mass reduction of about 10 percent or more, e.g. about 50 percent or more, after a period of implantation of one day or more, e.g. about 60 days or more, about 180 days or more, about 600 days or more, or 1000 days or less. In embodiments, the device exhibits fragmentation by erosion processes. The fragmentation occurs as, e.g., some regions of the device erode more rapidly than other regions. The faster eroding regions become weakened by more quickly eroding through the body of the endoprosthesis and fragment from the slower eroding regions. The faster eroding and slower eroding regions may be random or predefined. For example, faster eroding regions may be predefined by treating the regions to enhance chemical reactivity of the regions. Alternatively, regions may be treated to reduce erosion rates, e.g., by using coatings. In embodiments, only portions of the device exhibit erodibilty. For example, an exterior layer or coating may be erodible, while an interior layer or body is non-erodible. In embodiments, the endoprosthesis is formed from an erodible material dispersed within a non-erodible material such that after erosion, the device has increased porosity by erosion of the erodible material.

Erosion rates can be measured with a test device suspended in a stream of Ringer's solution flowing at a rate of 0.2 m/second. During testing, all surfaces of the test device can be exposed to the stream. For the purposes of this disclosure, Ringer's solution is a solution of recently boiled distilled water containing 8.60 gram sodium chloride, 0.30 gram potassium chloride, and 0.33 gram calcium chloride per liter.

Aspects and/or embodiments may have one or more of the following advantages. The endoprosthesis can be configured to deliver a therapeutic agent. The endoprosthesis can have surfaces that support cellular growth (endothelialization). The endoprosthesis, e.g., a drug eluting bioerodible stent, can include a large number of porous bioerodible capsules adhered to or embedded in the bioerodible stent. The capsules allow increase of drug-loading capacity of the stent. The drug elution profile over time can be controlled by selecting bioerodible metal that forms the capsules, porosity of the capsule walls, and the thickness of the capsule walls. The porosity of capsule walls, can be controlled, e.g., increased, by lowering the kinetic energy of the nanoclusters that impact the stent. The enhanced porosity facilitates drug loading. The wall thickness of capsules can be selected to control both drug eluting rate and erosion rates of the capsules and the stent. For example, capsules made of the same bioerodible metal with thicker walls may release drugs at a relatively slower rate than the ones with thinner walls. Thus, introducing capsules with different metal, different size, different wall thickness, or a geometry allows building a variety of drug release profiles. The endoprosthesis can be configured to erode in a predetermined fashion and/or at a predetermined time after implantation into a subject, e.g., a human subject. For example, the predetermined manner of erosion can be from an inside of the endoprosthesis to an outside of the endoprosthesis, or from a first end of the endoprosthesis to a second end of the endoprosthesis. The endoprosthesis may have portions which are protected from contact with bodily materials until it is desired for such portions to contact the bodily materials. The endoprosthesis may not need to be removed from the body after implantation. Lumens implanted with such endoprosthesis can exhibit reduced restenosis. The endoprosthesis is substantially free of polymer, therefore reducing the likelihood of any negative effects that may be caused by polymers. The endoprosthesis can have a low thrombogenecity.

Other aspects, features, objects, and advantages of the invention will be apparent from the description and drawings, and from the claims.

### DESCRIPTION OF DRAWINGS

FIGS. 1A-1C are longitudinal cross-sectional views illustrating delivery of a stent in a collapsed state, expansion of the stent, and deployment of the stent, respectively.
FIG 2 is a perspective view of a stent.
FIG 3 is a partial cross-section of a stent having drug-containing capsules attached to its surface.
FIG. 3A is a cross-sectional view of a drug-containing capsule.
FIG 3B is a somewhat diagrammatic side view of a stent with drug-containing capsules located at or near the stent surface.
FIG 3C is a somewhat diagrammatic side view of a stent with drug-containing capsules attached to the stent surface.
FIGS. 4A - 4C are longitudinal cross-sectional views of an endoprosthesis in a body lumen over time.
FIG. 5A-5F are cross-sectional views illustrating an embodiment of forming a stent.
FIG 6A-6B are cross-sectional views illustrating another embodiment of forming a stent.

Like reference symbols in the various drawings indicate like elements.

### DETAILED DESCRIPTION

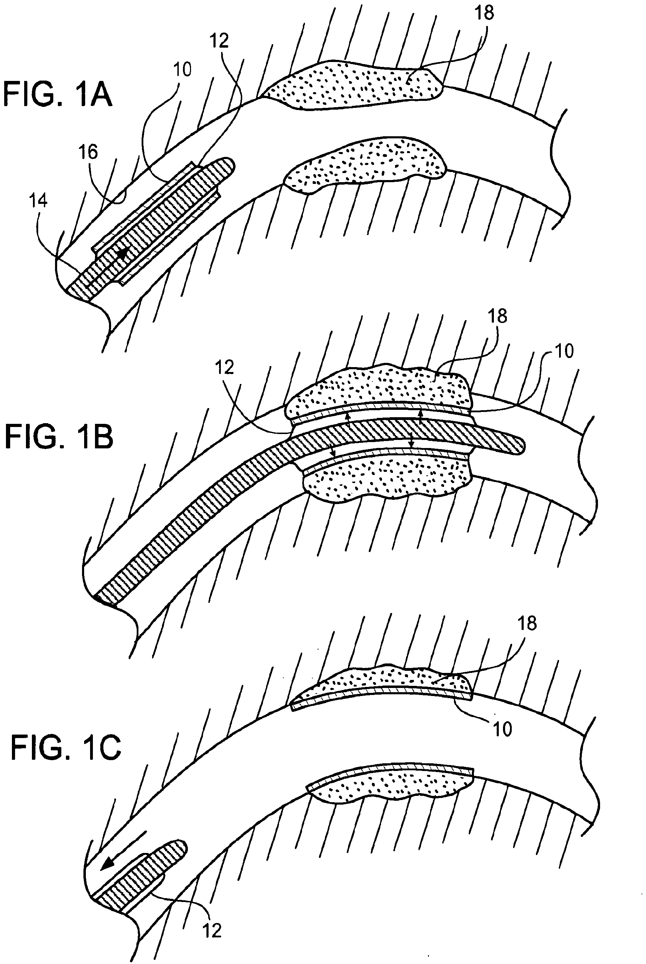

Referring to FIGS. 1A-1C, a bioerodible stent 10 is placed over a balloon 12 carried near a distal end of a catheter 14, and is directed through a lumen 16 (FIG 1 A) until the portion carrying the balloon 12 and stent 10 reaches the region of an occlusion 18. The stent 10 is then radially expanded by inflating the balloon and compressed against the vessel wall with the result that occlusion is compressed, and the vessel wall surrounding it undergoes a radial expansion (FIG 1 B). The pressure is then released from the balloon 12 and the catheter 14 is withdrawn from the vessel (FIG 1C), leaving behind the expanded stent 10 in lumen 16.

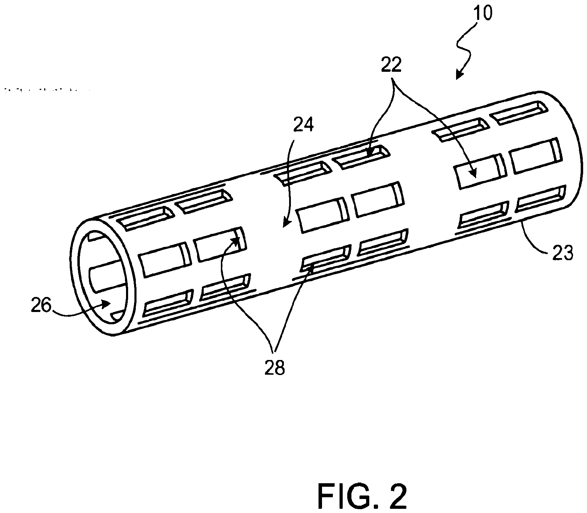

Referring to FIG. 2, the stent 10 includes a plurality of fenestrations 22 defined in a wall 23. Stent 10 includes several surface regions, including an outer, or abluminal, surface 24, an inner, luminal, surface 26, and a plurality of cutface surfaces 28. The stent can be balloon expandable, as illustrated above, or a self-expanding stent. Examples of stents are described in Heath '721, *supra.*

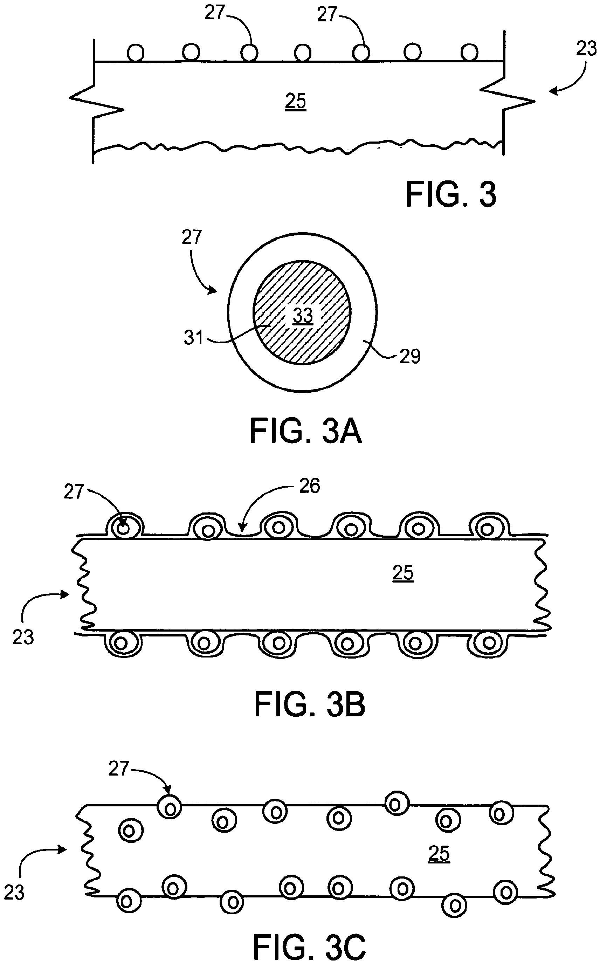

Referring to FIG. 3, a greatly enlarged cross-sectional view, a stent wall 23 includes a stent body 25 formed of a bioerodible material, e.g. a metal or an alloy, such as, e.g., defined in U.S. Patent Application Publication Nos. 2006/0052863 A1 and 2006/0052864 A1, both to Harder et al., and includes a plurality of capsules 27 on a surface of the stent, e.g., the abluminal side and/or other surfaces of the stent. Referring to Fig. 3A, a cross-section through a single capsule, the capsule 27 is preferably formed of a bioerodible material 29, e.g., a metal, defining a cavity 31 within which a drug 33 is contained. The wall of the capsule 27 can have interconnected porous structure (not shown) that allows drug loading to and eluting from the interior of the capsule. The pore size and porosity of the capsule wall may be controlled to regulate drug release rate as well as to regulate capsule erosion rate. More detail follows. The average pore size of the capsule wall can be selected from about 0.5nm to about 25nm, or fully closed capsules can be selected whereby the drug is only released if the shell is partly degraded

In embodiments, the bioerodible capsules, e.g., metal hollow spheres 27 can be attached to the stent body 25, e.g. by an encapsulating binder coating 26 (FIG. 3B), e.g. the metal hollow spheres can be attached to a surface of the stent through, e.g., chemical bonds such as metal-metal bonds, or a tie layer; or the metal hollow spheres 27 may embedded in or within, the bioerodible stent body 25, e.g., near the stent surface (FIG. 3C), e.g. the metal hollow spheres can also be incorporated into groves, pits, void spaces, and other features of the stent and the stent surface.

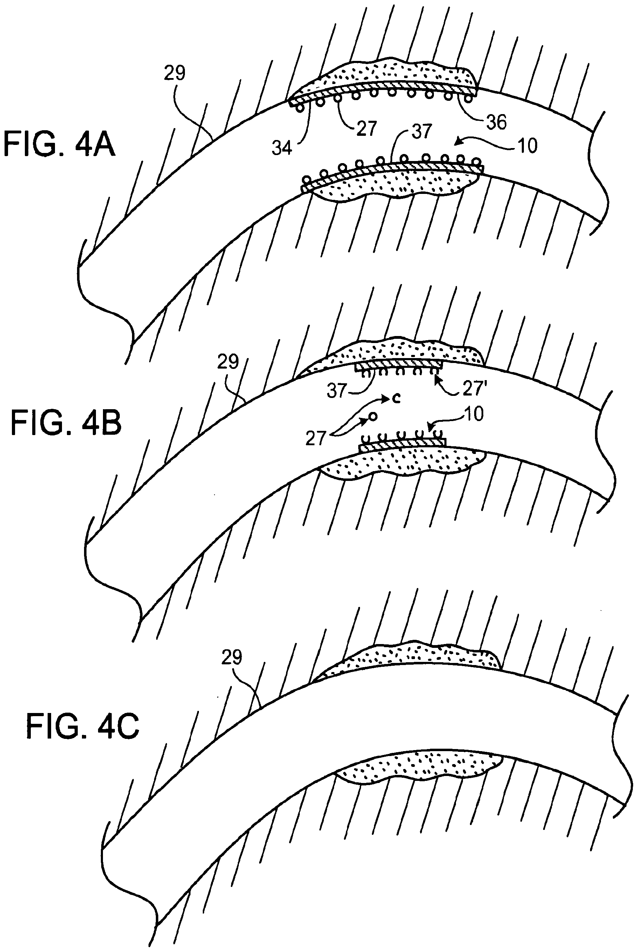

Referring to FIGS. 4A-4C, the stent 10 including the capsules 27 erodes over a period of time. For example, in embodiments, the stent and/or capsules exhibit substantial mass reduction, e.g., more than 20%, more than 50%, or more than 80% of mass reduction, over a period of time, until a function of the stent or the capsules, such as support of the lumen wall and/or drug delivery, is no longer needed or desirable. For example, a stent 10 implanted in a body vessel 29 can be configured to erode progressively from its end portions 34, 36 toward its middle segment 37, or from one end portion 34 toward the other end portion 36, which can, for example, enable the stent to maintain patency of a body lumen for a more prolonged period of time, and/or allow increased endothelialization of the endoprosthesis. During the erosion process of stent 10, referring particularly FIG. 4B, some bioerodible capsules 27 attached to the stent lose portions of the bioerodible shell (shown as 27') and release drugs contained in the cavity of the capsules into the surrounding environment while the other capsules may be dislodged and released into the body vessel, for example, capsules that were originally attached to end portions 34 and 36. The dislodged capsules may further erode and eventually release the encapsulated drug (see above). These capsules can be made fully closed by starting with a porous capsule, filling it with a drug, after which a second metal layer is deposited on the outer layer. Bioerodible stents are also disclosed in, e.g., commonly assigned U. S. Patent Application Serial Nos. 11/327,149, filed January 15, 2006 (U.S. Patent Application Publication No. 2007-0156231), and 11/355,368, filed February 16, 2006 (U.S. Patent Application Publication No. 2007-0191931), and Provisional Application No. 60/845,341, filed September 18, 2006 (corresponding to U.S. Patent Application Publication No. 2008-0071350) [Attorney Docket Nos. 10527-656001, 10527-730001, and 10527-712P01].

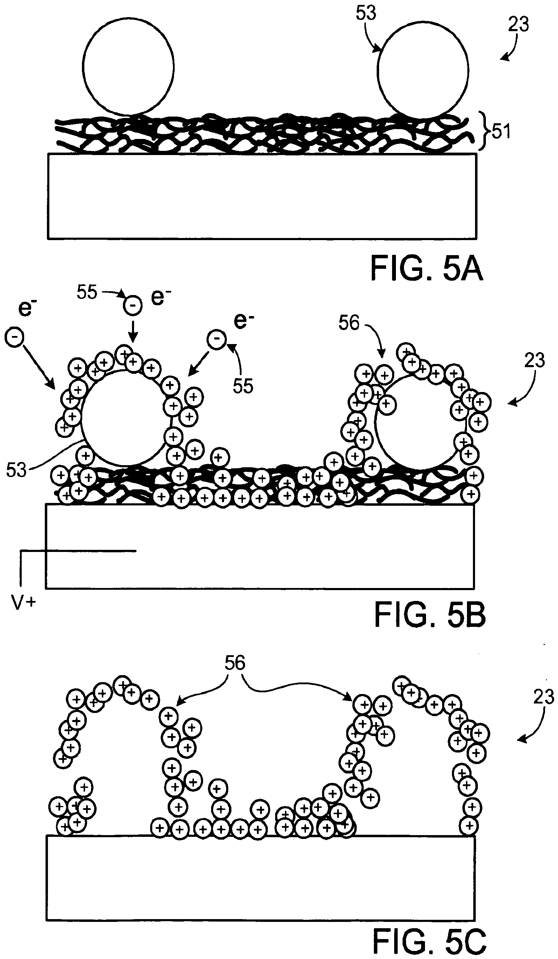

Referring to FIGS. 5A-5F, greatly enlarged cross-sectional views of a stent wall 23 at different stages of a particular method of forming a bioerodible stent are shown. The method includes applying sacrificial polymeric templates, e.g., ionic nanosized latex particle spheres, and a polymeric binding layer to a stent surface; depositing metal nanoclusters over the templates and the binding layer; removing the sacrificial templates and the binding layer to form hollow metallic capsules having porous walls attached to the stent surface; and loading the capsules with a therapeutic agent or drug.

Referring particularly to FIG 5A, templating spheres 53, such as polystyrene micro- or nano-spheres, and a polymeric binding layer are applied to a surface of the stent wall 23, e.g., the abluminal surface, via a self-assembly process such as a layer-by-layer ("LBL") technique in which the layers, e.g., polyelectrolytes 51 and spheres 53, electrostatically self-assemble. In the LBL technique, a first layer having a first net surface charge is deposited on an underlying substrate eventually activated by means of a plasma process, followed by a second layer having a second net surface charge that is opposite in sign to the net surface charge of the first layer. Thus, the charge on the outer layer is reversed upon deposition of each sequential layer. Additional first and second layers can then be deposited on the substrate in alternating fashion to build multi-layered structure to a predetermined or targeted thickness. For example, in FIG. 5A, deposited polyelectrolyte layer 51 can either be a polyelectrolyte monolayer or polyelectrolyte multilayers ("PEM") with thickness ranging from 0.3 nm to 1 µm, e.g., 1 nm to 500 nm. The thickness can be selected by the necessary charge density required to deposit the sacrificial template. On top of the polyelectrolyte layer 51, charged sacrificial templates 53, e.g., polystyrene spheres, polymethylmethacrylate ("PMMA"), or silica templates, with net surface charges opposite in sign to those of layer 51, are deposited.

In certain embodiments, the LBL assembly can be conducted by exposing a selected charged substrate (e.g., stent) to solutions or suspensions that contain species of alternating net charge, including solutions or suspensions that contain charged templates (e.g., polystyrene spheres), polyelectrolytes, and, optionally, charged therapeutic agents. The concentration of the charged species within these solutions and suspensions, which can be dependent on the types of species being deposited, can range, for example, from about 0.01 mg/mL to about 100 mg/mL (or to about 50 mg/mL, or to about 30 mg/mL). The pH of these suspensions and solutions can be such that the templates, polyelectrolytes, and optional therapeutic agents maintain their charge. Buffer systems can be used to maintain charge. The solutions and suspensions containing the charged species (e.g., solutions/suspensions of templates, polyelectrolytes, or other optional charged species such as charged therapeutic agents) can be applied to the charged substrate surface using a variety of techniques. Examples of techniques include spraying techniques, dipping techniques, roll and brush coating techniques, techniques involving coating via mechanical suspension such as air suspension, ink jet techniques, spin coating techniques, web coating techniques and combinations of these processes. Layers can be applied over an underlying substrate by immersing the entire substrate (e.g., stent) into a solution or suspension containing the charged species, or by immersing half of the substrate into the solution or suspension, flipping the same, and immersing the other half of the substrate into the solution or suspension to complete the coating. In some embodiments, the substrate is rinsed after application of each charged species layer, for example, using a washing solution with a pH that maintains the charge of the outer layer.

Suitable sacrificial templates include polymeric spheres, silica spheres, nanoparticles, carbon fibers, polymeric fibers, or carbon nanotubes. The sacrificial templates described herein can have selected shapes, e.g., shapes of a sphere, a cube, a rod, a fiber, a spindle, a tube, etc. Sacrificial templates are templates capable of being removed once a desired configuration, often complementary to the shape of the templates, is achieved. In some embodiments, the templates are completely removed once the complementary configurations are created. In other embodiments, the templates are at least partially maintained in the final product. For example, carbon fibers and carbon nanotubes can be kept within the final configurations as a reinforcing component. In a particular embodiment, spherical sacrificial templates, e.g., polystyrene spheres, are used. When polystyrene spheres are applied as templates, a structure with spherical cavities or a hollow structure will be obtained after the polystyrene spheres are removed. In embodiments, polystyrene spheres have a diameter of about 10 nm to 10 µm, e.g., about 20 nm to 500 nm. Suitable polystyrene spheres are available from Microparticles Gmbh, Research and Development Laboratory, Volmerstr. 9A, UTZ, Geb.3.5.1, D-12489 Berlin. In particular embodiments, sacrificial templates can be pretreated with polyelectrolytes via LBL assembly to have a PEM coating. Next, the PEM-coated templates can be suspended or dissolved in a solvent and be applied to a substrate. When PEM-coated templates are used, layer 51 is optional.

Polyelectrolytes are polymers having charged (e.g., ionically dissociable) groups. The number of these groups in the polyelectrolytes can be so large that the polymers are soluble in polar solvents (including water) when in ionically dissociated form (also called polyions). Depending on the type of dissociable groups, polyelectrolytes can be classified as polyacids and polybases. When dissociated, polyacids form polyanions, with protons being split off. Polyacids include inorganic, organic, and biopolymers. Examples of polyacids are polyphosphoric acids, polyvinylsulfuric acids, polyvinylsulfonic acids, polyvinylphosphonic acids, and polyacrylic acids. Examples of the corresponding salts, which are called polysalts, are polyphosphates, polyvinylsulfates, polyvinylsulfonates, polyvinylphosphonates, and polyacrylates. Polybases contain groups that are capable of accepting protons, e.g., by reaction with acids, with a salt being formed. Examples of polybases having dissociable groups within their backbone and/or side groups are polyallylamine, polyethylimine, polyvinylamine, and polyvinylpyridine. By accepting protons, polybases form polycations. Some polyelectrolytes have both anionic and cationic groups, but nonetheless have a net positive or negative charge.

The polyelectrolytes can include those based on biopolymers. Examples include alginic acid, gum arabicum, nucleic acids, pectins, and proteins, chemically modified biopolymers such as carboxymethyl cellulose and lignin sulfonates, and synthetic polymers such as polymethacrylic acid, polyvinylsulfonic acid, polyvinylphosphonic acid, and polyethylenimine. Linear or branched polyelectrolytes can be used. Using branched polyelectrolytes can lead to less compact polyelectrolyte multilayers having a higher degree of wall porosity. In some embodiments, polyelectrolyte molecules can be crosslinked within or/and between the individual layers, to enhance stability, e.g., by crosslinking amino groups with aldehydes. Furthermore, amphiphilic polyelectrolytes, e.g., amphiphilic block or random copolymers having partial polyelectrolyte character, can be used in some embodiments to affect permeability towards polar small molecules.

Other examples of polyelectrolytes include low-molecular weight polyelectrolytes (e.g., polyelectrolytes having molecular weights of a few hundred Daltons up to macromolecular polyelectrolytes (e.g., polyelectrolytes of synthetic or biological origin, which commonly have molecular weights of several million Daltons). Still other examples of polyelectrolyte cations (polycations) include protamine sulfate polycations, poly(allylamine) polycations (e.g., poly(allylamine hydrochloride) (PAH)), polydiallyldimethylammonium polycations, polyethyleneimine polycations, chitosan polycations, gelatin polycations, spermidine polycations, and albumin polycations. Examples of polyelectrolyte anions (polyanions) include poly(styrenesulfonate) polyanions (e.g., poly(sodium styrene sulfonate) (PSS)), polyacrylic acid polyanions, sodium alginate polyanions, eudragit polyanions, gelatin polyanions, hyaluronic acid polyanions, carrageenan polyanions, chondroitin sulfate polyanions, and carboxymethylcellulose polyanions.

Still more examples of polyelectrolytes include poly(ethyleneimine (PEI), poly(allylamine hydrochloride) (PAH), and polyacrylic acid, and other polyelectrolytes and LBL assembly are disclosed in commonly assigned U.S. Patent Application Serial No. 10/985,242 (U.S. Patent Application Publication No. 2006-0100696).

Referring particularly to FIG 5B, negatively charged metallic nanoclusters 55 formed of, e.g., bioerodible metal such as magnesium, iron, calcium, aluminum, tungsten, or alloys thereof are deposited on top of the templating particles 53 and the stent surface in an acceleration electrical field. The stent can be rotated (or wobbled), or, alternatively, the electric field that accelerates the nanoparticles can as well be changed dynamically in direction and strength by use of deflectors, such that the nanoclusters hit the templating particles and stent from all directions, as illustrated by the single arrows, therefore the metal coating 56 deposited on the templating particles has substantially uniform thickness.

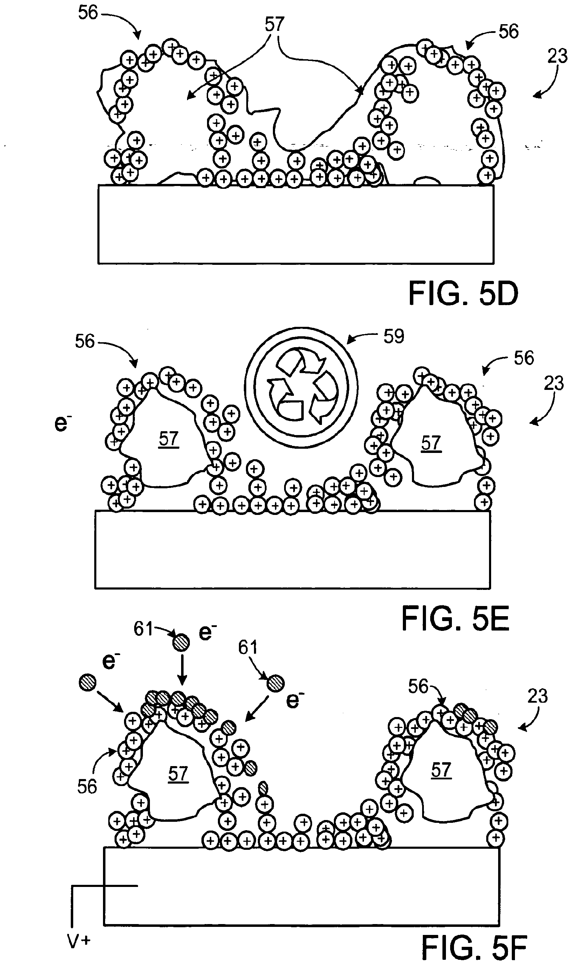

A suitable nanocluster deposition system is available from, e.g., Mantis Deposition Ltd., England (http://www.mantisdeposition.com), in which ionized metal nanoclusters, such as copper, gold, ruthenium, or stainless steel, can be produced by magnetron sputtering followed by thermalization and condensation in relatively high pressure zones (e.g., about 0.1 to a few millibars) and accelerated towards a substrate, e.g., a stent, by an applied electric field. Most of the nanoclusters generated are charged and may therefore be mass selected by a linear quadrupole or a mass filter and selectively deposited on the substrate. For example, typical diameter of the nanoclusters generated can be about 0.7 to 20nm and size distribution of the nanoclusters can be selectively narrowed to about +/-20%, or even to +/-2%. The kinetic energy (e.g., about 10 eV to 10 keV) of the nanoclusters partly controlled by the applied electric field may be selected to induce nanoclusters to partially melt upon impact with the substrate and other nanoclusters, and thus adhere to, e.g. fuse with, the substrate and fuse with other clusters to form a porous metallic coating. The pore size and porosity of the porous coating can be controlled by both the size of the nanoclusters and their kinetic energy. Generally, higher kinetic energy renders the metal coating less porous. In embodiments, a first collection of larger nanoclusters with a diameter of, e.g., 100 nm are deposited onto the templating particles. The voltage (shown as V+) can be controlled high enough for the nanoclusters to penetrate through the polyelectrolyte layer 51 and adhere to the stent surface. Generally, the larger the nanoclusters, the larger the pores of the deposited metal coating, and the easier the drug loading into the metallic capsules. In embodiments, a second collection of nanoclusters with smaller diameter can later be deposited on top of the first collection to form a top layer with smaller pores, further regulating drug release, as illustrated in FIG 5F. Nanoparticle deposition is further disclosed by Weber et al., U.S. Provisional Application No. 60/857,849, filed November 9, 2006 (corresponding to U.S. Patent Application Publication No. 2008-0147177) [BSC Docket No. 06-01579], A. H. Kean, Mantis Deposition Ltd., NSTI Nano Tech 2006, Boston, May 7th-11th 2006, and in Y. Qiang, Surface and Coating Technology, 100-10 1, 27-32 (1998).

As a next step (illustrated in FIG 5C), the templating particles 53 as well as polyelectrolyte layer 51 are removed by, e.g., heat, UV light exposure, plasma, or solution based methods, leaving porous hollow metallic capsules 56 on the stent surface. Referring particularly to FIG. 5D, in embodiments, a pharmaceutically active agent or drug 57 can be loaded into the cavities of the capsules 56. The drug can be loaded by repeated cycles of soaking and drying the stent with the metallic capsules. In embodiments, conditions may be applied to facilitate drug loading, e.g., a supercritical fluid can be applied as a solvent during this process or vacuum can be used initially to remove air from the cavities. For example, a drug can be loaded first using a solvent, e.g., a super critical fluid (carbon dioxide with a co-solvent, ethanol, of 10% by weight), which penetrates the porous walls of the capsules. Referring to FIG 5E, when the loading process results in drug deposition both inside and outside the capsules, the drug deposited outside can be removed by a mechanism 59, such as an ablating laser process with energy selected to remove only drugs on the outside surface of the coating, or briefly rinsing the stent with a solvent that does not penetrate the metallic capsule walls easily but dissolves the drug, e.g., dimethyl sulfoxide (DMSO). In some embodiments, the drug can be loaded within the pores of the capsule walls. The stent can further include more than one therapeutic agent by having more than one drug within each capsule, or by having different drugs in different capsules. Optionally, as illustrated in FIG 5F and discussed above, by rotating the stent in a nanocluster deposition system, a second layer of finer nanoclusters 61 can be deposited over the metallic capsules 56 to further regulate drug release through smaller pores. Alternatively, a higher acceleration voltage can be applied to, e.g., nanoclusters of size similar to those forming the capsules, creating a less porous top layer over the metallic capsules to, e.g., decrease drug release rate and/or erosion rate of the metallic capsules and stent.

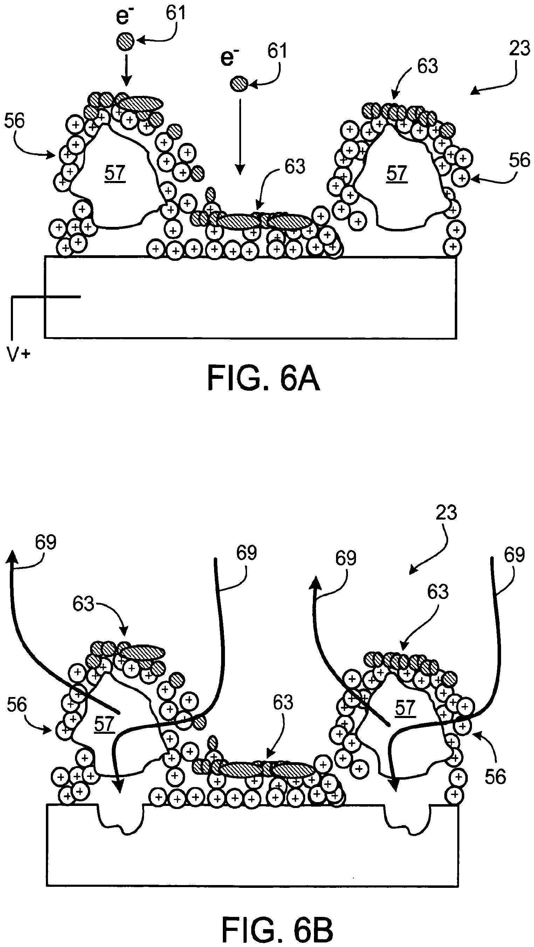

The stent, formed ofbioerodible metal, can have specific erosion profile by controlling parameters of the nanocluster deposition process. For example, referring to FIG 6A, the stent coated with drug-loaded metallic capsules 56 is fixed at a straight angle to the velocity direction (illustrated as single arrows) of the second collection of nanoclusters 61, therefore, a noncontinuous film 63 is formed. The kinetic energy of the nanoclusters 61 is selected to be high enough to form a substantially nonporous top film 63 over porous capsules 56 and in spaces between neighboring capsules. As a result, referring to FIG. 6B, fluid 69 is only able to penetrate through the sides of the capsules 56, eroding the stent surface underneath the capsules to form pits, while the surface in between the capsules is at least temporarily protected by the nonporous layer 63.

In other embodiments, a collection of hollow metallic capsules can be formed first and then loaded with drugs by, e.g., dipping, spraying, brushing, rolling, or repeated cycles of soaking and drying the capsules in a solution containing the selected drug. The drug-loaded metallic capsules once formed, can be mixed with a biodegradable matrix, such as glucose, and applied to the stent surface. Hollow metallic capsules can be formed using sacrificial templates, e.g., polymer spheres. As an example, hollow metallic spheres composed of nickel, cobalt, or nickel-cobalt alloy are obtained by first forming uniform and stable core-shell microspheres composed of a poly(methyl methacrylate) (PMMA) core and a thin metallic shell of nickel-phosphorus, cobalt-phosphorus, or mixed metal alloys (CoNiP, NiFeP, CoFeP). The core-shell microspheres can be prepared by dispersion polymerization of methyl methacrylate followed by electroless plating to form the metal/alloy shell. The thickness of the metal/alloy shell can be precisely tuned by adjusting the immersion time of the polymer microspheres in the electroless plating bath. In some embodiments, depending on the deposited metallic material, various properties, such as magnetic properties, from paramagnetic to ferromagnetic, can be achieved. Finally, uniform hollow metallic spheres composed of metal or alloy are obtained by dissolving the polymer core. Additional information can be found, for example, in "Using Electroless Deposition for the Preparation of Micron Sized Polymer/Metal Core/Shell Particles and Hollow Metal Spheres," Tierno et al., J. Phys. Chem. B, 110, 3043-3050 (2006).

In embodiments, bioerodible metal nanoclusters and capsules are adhered only on the abluminal surface of the stent. This construction may be accomplished by, e.g. coating the stent before forming the fenestrations. In other embodiments, bioerodible metal nanoclusters and capsules are adhered only on abluminal and cutface surfaces of the stent. This construction may be accomplished by, e.g., coating a stent containing a mandrel that shields the luminal surfaces. Masks can be used to shield selected portions of the stent. In embodiments, the stent metal is bioerodible, including one or more of a metallic component (e.g., a metal or alloy). Bioerodible materials are described, for example, in commonly assigned U. S. Patent Application Serial Nos. 11/327,149 (U.S. Patent Application Publication No. 2007-0156231), 11/355,368 (U.S. Patent Application Publication No. 2007-0191931), and U.S. Provisional Application No. 60/845,341 (corresponding to U.S. Patent Application Publication No. 2008-0071350), *supra*, as well as in U.S. Patent No. 6,287,332 to Bolz; U.S. Patent Application Publication No. 2002/0004060 A1 to Heublein; U.S. Patent Nos. 5,587,507 and 6,475,477 to Kohn et al*.* Examples of bioerodible metals include alkali metals, alkaline earth metals (*e.g.*, magnesium), iron, zinc, and aluminum. Examples of bioerodible metal alloys include alkali metal alloys, alkaline earth metal alloys (*e.g.*, magnesium alloys), iron alloys (*e.g*., alloys including iron and up to seven percent carbon), and zinc alloys. Examples of bioerodible non-metals include bioerodible polymers, such as, e.g., polyanhydrides, polyorthoesters, polylactides, polyglycolides, polysiloxanes, cellulose derivatives and blends or copolymers of any of these.

In other embodiments, the stent can include one or more biostable materials in addition to one or more bioerodible materials. For example, the bioerodible material may be provided as a coating on a biostable stent body. Examples of biostable materials include stainless steel, tantalum, nickel-chrome, cobalt-chromium alloys such as Elgiloy^{®} and Phynox^{®}, Nitinol (*e.g.*, 55% nickel, 45% titanium), and other alloys based on titanium, including nickel titanium alloys and thermo-memory alloy materials. Stents including biostable and bioerodible regions are described, for example, in U.S. Patent Application Serial No. 11/004,009, filed on December 3, 2004, and entitled "Medical Devices and Methods of Making the Same" (U.S. Patent Application Publication No. 2006-0122694). The material can be suitable for use in, for example, a balloon-expandable stent, a self-expandable stent, or a combination of both (see e.g., U.S. Patent No. 5,366,504).

The terms "therapeutic agent," "pharmaceutically active agent," "pharmaceutically active material," "pharmaceutically active ingredient," "drug," and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. By small organic molecule is meant an organic molecule having 50 or fewer carbon atoms, and fewer than 100 non-hydrogen atoms in total.

Exemplary therapeutic agents include, e.g., anti-thrombogenic agents (e.g., heparin); anti-proliferative/anti-mitotic agents (e.g., paclitaxel, 5-fluorouracil, cisplatin, vinblastine, vincristine, inhibitors of smooth muscle cell proliferation (e.g., monoclonal antibodies), and thymidine kinase inhibitors); antioxidants; anti-inflammatory agents (e.g., dexamethasone, prednisolone, corticosterone); anesthetic agents (e.g., lidocaine, bupivacaine, and ropivacaine); anti-coagulants; antibiotics (e.g., erythromycin, triclosan, cephalosporins, and aminoglycosides); and agents that stimulate endothelial cell growth and/or attachment. Therapeutic agents can be nonionic, or they can be anionic and/or cationic in nature. Therapeutic agents can be used singularly, or in combination. Preferred therapeutic agents include inhibitors of restenosis (e.g., paclitaxel), antiproliferative agents (e.g., cisplatin), everolimus, and antibiotics (e.g., erythromycin). Additional examples of therapeutic agents are described in U.S. Patent Application Publication No. 2005/0216074 A1. Polymers for drug elution coatings are also disclosed in U.S. Patent Application Publication No. 2005/019265 A1. A functional molecule, e.g. an organic, drug, polymer, protein, DNA, and similar material, can be incorporated into groves, pits, void spaces, and other features of the stent.

Any stent described herein can be dyed or rendered radio-opaque by addition of, e.g., radio-opaque materials such as barium sulfate, platinum, or gold, or by coating with a radio-opaque material.

Any of the stents described herein or any portion of any stent described herein can be coated with a bioerodible material or a non-bioerodible material. For example, the coating can be used to deliver a drug, to protect a portion of the stent, to reduce uncontrolled fragmentation, and/or to prevent contact between the stent or a portion of a stent and a portion of a lumen.

The stents described herein can be configured for vascular, e.g. coronary and peripheral vasculature or non-vascular lumens. For example, they can be configured for use in the esophagus or the prostate. Other lumens include biliary lumens, hepatic lumens, pancreatic lumens, and urethral lumens.

The stent can be of a desired shape and size (e.g., coronary stents, aortic stents, peripheral vascular stents, gastrointestinal stents, urology stents, tracheal/bronchial stents, and neurology stents). Depending on the application, the stent can have a diameter of between, e.g., about 1 mm to about 46 mm. In certain embodiments, a coronary stent can have an expanded diameter of from about 2 mm to about 6 mm. In some embodiments, a peripheral stent can have an expanded diameter of from about 4 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. The stent can be balloon-expandable, self-expandable, or a combination of both (e.g., U.S. Patent No. 6,290,721).

The processes or method described herein can be performed on other endoprostheses or medical devices, such as a stent precursor, e.g. metal tube, catheters, guide wires, and filters.

Still other embodiments are in the following claims.

## Claims

1. A method of coating an endoprosthesis, the method comprising:
applying a polyelectrolyte layer (51) to a surface,
applying a sacrificial template (53) over the polyelectrolyte layer (51);
applying a plurality of metal nanoclusters (55) over the template (53);
removing the sacrificial template (53) to form a cavity defined by the metal nanoclusters (55); and
loading a drug (57) into the cavity.

2. The method of claim 1, further comprising applying the plurality of metal nanoclusters comprising bioerodible metal.

3. The method of claim 1, further comprising removing the polyelectrolyte layer and the sacrificial template by heating, UV light exposure, or dissolution.

4. The method of claim 1, comprising applying the polyelectrolyte layer and the sacrificial template by LBL deposition.

5. The method of claim 1, wherein the sacrificial template is a polymer particle, wherein the polymer particle has a size of 10 nm to 10 µm.

6. The method of claim 1, wherein the sacrificial template is a sphere.

7. An endoprosthesis, comprising:
a body comprising a first bioerodible metal and a surface, and
one or more capsules (27, 56) formed of a second bioerodible metal disposed on the surface, wherein the capsule (27, 56) comprises at least one porous peripheral region, and a drug-containing core (33, 57), wherein the capsules are obtainable by a method comprising applying a polyelectrolyte layer (51) to a surface,
applying a sacrificial template (53) over the polyelectrolyte layer (51);
applying a plurality of metal nanoclusters (55) over the template (53);
removing the sacrificial template (53) to form a cavity defined by the metal nanoclusters (55).

8. The endoprosthesis of claim 7, wherein the first metal is selected from among magnesium, iron, zinc, aluminum, calcium, tungsten, and alloys thereof, and wherein the second metal is selected from among magnesium, iron, zinc, aluminum, calcium, tungsten, and alloys thereof.

9. The endoprosthesis of claim 7 or 8, wherein the first and second bioerodible metals are the same.

10. The endoprosthesis of claim 7, wherein the endoprosthesis is free of polymer.

11. The endoprosthesis of claim 7, wherein the porous peripheral region of the capsule comprises a plurality of interconnected pores having a width of 0.5 nm to about 100 nm.

12. The endoprosthesis of claim 7, wherein the porous peripheral region has a porosity of 70% or less.

13. The endoprosthesis of claim 7, wherein the capsule has the shape of a hollow sphere, wherein the hollow sphere has an outer diameter of 20 nm to 10 µm, and wherein the hollow sphere has an inner diameter of 10 nm to 5 µm.

14. The endoprosthesis of claim 7, wherein the porous peripheral region has a thickness of 1 nm to 1 µm.

## Patentansprüche

1. Verfahren zum Beschichten einer Endoprothese, das Verfahren umfassend:
Aufbringen einer Polyelektrolytschicht (51) an eine Oberfläche;
Aufbringen einer Opfermatrize (53) über die Polyelektrolytschicht (51);
Aufbringen einer Vielzahl von Metall-Nanoclustern (55) über die Matrize (53);
Entfernen der Opfermatrize (53) um einen Hohlraum zu formen, der von den Metall-Nanoclustern (55) geformt wird; und
Laden eines Wirkstoffs (57) in den Hohlraum

2. Das Verfahren nach Anspruch 1, weiter umfassend Aufbringen einer Vielzahl von Metall-Nanoclustern umfassend bioerodierbares Metall.

3. Das Verfahren nach Anspruch 1, weiter umfassend Entfernen der Polyelektrolytschicht und der Opfermatrize durch Erwärmen, Bestrahlung mit UV-Licht, oder Auflösen.

4. Das Verfahren nach Anspruch 1, weiter umfassend Aufbringen der Polyelektrolytschicht und der Opfermatrize durch LBL Niederschlag.

5. Das Verfahren nach Anspruch 1, wobei die Opfermatrize ein Polymerpartikel ist, wobei das Polymerpartikel eine Größe von 10 nm bis 10 µm hat.

6. Das Verfahren nach Anspruch 1, wobei die Opfermatrize eine Kugel ist.

7. Eine Endoprothese umfassend:
ein Körper umfassend ein erstes bioerodierbares Metall und eine Oberfläche, und
eine oder mehrere Kapseln (27, 56) geformt aus einem auf die Oberfläche angeordneten zweiten bioerodierbaren Metall, wobei die Kapsel (27, 56) zumindest eine poröse periphere Region und einen Wirkstoffbeinhaltenden Kern (33, 57) umfasst,
wobei die Kapseln erhalten werden durch ein Verfahren umfassend:
Aufbringen einer Polyelektrolytschicht (51) an eine Oberfläche;
Aufbringen einer Opfermatrize (53) über die Polyelektrolytschicht (51);
Aufbringen einer Vielzahl von Metall-Nanoclustern (55) über die Matrize (53);
Entfernen der Opfermatrize (53) um einen Hohlraum zu formen, der von den Metall-Nanoclustern (55) geformt wird.

8. Die Endoprothese nach Anspruch 7, wobei das erste Metall ausgewählt ist aus Magnesium, Eisen, Zink, Aluminium, Kalzium, Wolfram, und Legierungen daraus, und wobei das zweite Metall ausgewählt ist aus Magnesium, Eisen, Zink, Aluminium, Kalzium, Wolfram, und Legierungen daraus.

9. Die Endoprothese nach Anspruch 7 oder 8, wobei das erste und zweite bioerodierbare Metall das gleiche sind.

10. Die Endoprothese nach Anspruch 7, wobei die Endoprothese frei von Polymer ist.

11. Die Endoprothese nach Anspruch 7, wobei die poröse periphere Region der Kapsel eine Vielzahl von miteinander verbundenen Poren umfasst, die eine Breite von 0,5 nm bis ungefähr 100 nm haben.

12. Die Endoprothese nach Anspruch 7, wobei die poröse periphere Region eine Porosität von 70% oder weniger hat.

13. Die Endoprothese nach Anspruch 7, wobei die Kapsel die Form einer hohlen Kugel hat, wobei die hohle Kugel einen äußern Durchmesser von 20 nm bis 10 µm hat und wobei die hohle Kugel einen inneren Durchmesser von 10 nm bis 5 µm hat.

14. Die Endoprothese nach Anspruch 7, wobei die poröse periphere Region eine Dicke von 1 nm bis 1 µm hat.

## Revendications

1. Procédé de revêtement d'une endoprothèse, le procédé comprenant :
l'application d'une couche de polyélectrolyte (51) sur une surface ;
l'application d'une matrice sacrificielle (53) par-dessus la couche de polyélectrolyte (51) ;
l'application d'une pluralité de nanoagrégats métalliques (55) par-dessus la matrice (53) ;
le retrait de la matrice sacrificielle (53) pour former une cavité définie par les nanoagrégats métalliques (55) ; et
l'introduction d'un médicament (57) dans la cavité.

2. Procédé selon la revendication 1, comprenant en outre l'application de la pluralité de nanoagrégats métalliques comprenant un métal bioérodable.

3. Procédé selon la revendication 1, comprenant en outre le retrait de la couche de polyélectrolyte et de la matrice sacrificielle par chauffage, exposition à un rayonnement UV, ou dissolution.

4. Procédé selon la revendication 1, comprenant l'application de la couche de polyélectrolyte et de la matrice sacrificielle par dépôt couche par couche.

5. Procédé selon la revendication 1, dans lequel la matrice sacrificielle est une particule polymère, la particule polymère ayant une taille de 10 nm à 10 µm.

6. Procédé selon la revendication 1, dans lequel la matrice sacrificielle est une sphère.

7. Endoprothèse, comprenant :
un corps comprenant un premier métal bioérodable et une surface ; et
une ou plusieurs capsules (27, 56) constituées d'un deuxième métal bioérodable disposées sur la surface, les capsules (27, 56) comprenant au moins une région périphérique poreuse, et un coeur contenant un médicament (33, 57),
les capsules pouvant être obtenues par un procédé comprenant l'application d'une couche de polyélectrolyte (51) sur une surface ;
l'application d'une matrice sacrificielle (53) par-dessus la couche de polyélectrolyte (51) ;
l'application d'une pluralité de nanoagrégats métalliques (55) par-dessus la matrice (53) ; et
le retrait de la matrice sacrificielle (53) pour former une cavité définie par les nanoagrégats métalliques (55).

8. Endoprothèse selon la revendication 7, dans laquelle le premier métal est choisi parmi le magnésium, le fer, le zinc, l'aluminium, le calcium, le tungstène et les alliages de ceux-ci, et dans laquelle le deuxième métal est choisi parmi le magnésium, le fer, le zinc, l'aluminium, le calcium, le tungstène et les alliages de ceux-ci.

9. Endoprothèse selon la revendication 7 ou 8, dans laquelle le premier et le deuxième métal bioérodable sont identiques.

10. Endoprothèse selon la revendication 7, l'endoprothèse étant exempte de polymère.

11. Endoprothèse selon la revendication 7, dans laquelle la région périphérique poreuse de la capsule comprend une pluralité de pores interconnectés ayant une largeur de 0,5 nm à environ 100 nm.

12. Endoprothèse selon la revendication 7, dans laquelle la région périphérique poreuse a une porosité de 70 % ou moins.

13. Endoprothèse selon la revendication 7, dans laquelle les capsules ont la forme d'une sphère creuse, la sphère creuse ayant un diamètre extérieur de 20 nm à 10 µm, et la sphère creuse ayant un diamètre intérieur de 10 nm à 5 µm.

14. Endoprothèse selon la revendication 7, dans laquelle la région périphérique poreuse a une épaisseur de 1 nm à 1 µm.
